# EUROPEAN PATENT APPLICATION

(11) **EP 0 670 369 A2**
(43) Date of publication of application: **06.09.1995**
(21) Application number: 95102829.9
(22) Date of filing: 28.02.1995
(51) Int. Cl.: C12N 15/12, C07K 14/47, C12P 21/02, C07K 16/18, A61K 38/17, A61K 39/395

(54) **A novel peptide related to human programmed cell death and DNA encoding it**

(30) Priority: 01.03.1994 JP 55224/94
(71) Applicant: ONO PHARMACEUTICAL CO., LTD., Osaka-shi Osaka (JP); Honjo, Tasuku, Sakyo-ku, Kyoto-shi, Kyoto (JP)
(72) Inventor: Honjo, Tasuku, Kyoto-shi, Kyoto (JP); Ishida, Yasumasa, Newton, Massachusetts 02164 (JP); Shinohara, Takashi, Sakyo-ku, Kyoto-shi, Kyoto (JP)
(74) Representative: Henkel, Feiler, Hänzel & Partner

(57) **Abstract**

A membrane protein related to human programmed cell death (PD-1) and DNA encoding the said protein is new. PD-1 protein may be useful for the treatment of various infections , immunological depression or acceleration, or tumour etc.

SEQUENCE LISTING

(1) GENERAL INFORMATION:

(i) APPLICANT:
(A) NAME: ONO PHARMACEUTICAL CO., LTD.
(B) STREET: 1-5, Doshomachi 2-chome
(C) CITY: Chuo-ku, Osaka-shi
(D) STATE: Osaka
(E) COUNTRY: Japan
(F) POSTAL CODE (ZIP): 541

(A) NAME: HONJO, TASUKU
(B) STREET: Kan'yuchi, Kitashirakawa Oiwakecho, Sakyo-ku
(C) CITY: Kyoto-shi
(D) STATE: Kyoto
(E) COUNTRY: Japan
(F) POSTAL CODE (ZIP): 606

(ii) TITLE OF INVENTION: A NOVEL PEPTIDE RELATED TO HUMAN PROGRAMMED
CELL DEATH AND DNA ENCODING IT

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO:1:

(i) SEQUENCE CHARACTERISTICS:
(A) LENGTH: 288 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:1:

(2) INFORMATION FOR SEQ ID NO:2:

    (i) SEQUENCE CHARACTERISTICS:
        (A) LENGTH: 864 base pairs
        (B) TYPE: nucleic acid
        (C) STRANDEDNESS: single
        (D) TOPOLOGY: linear

    (ii) MOLECULE TYPE: cDNA to mRNA

(xi) SEQUENCE DESCRIPTION: SEQ ID NO:2:

ATGCAGATCC CACAGGCGCC CTGGCCAGTC GTCTGGGCGG TGCTACAACT GGGCTGGCGG 60

CCAGGATGGT TCTTAGACTC CCCAGACAGG CCCTGGAACC CCCCCACCTT CTCCCCAGCC 120

CTGCTCGTGG TGACCGAAGG GGACAACGCC ACCTTCACCT GCAGCTTCTC CAACACATCG 180

GAGAGCTTCG TGCTAAACTG GTACCGCATG AGCCCCAGCA ACCAGACGGA CAAGCTGGCC 240

GCCTTCCCCG AGGACCGCAG CCAGCCCGGC CAGGACTGCC GCTTCCGTGT CACACAACTG 300

CCCAACGGGC GTGACTTCCA CATGAGCGTG GTCAGGGCCC GGCGCAATGA CAGCGGCACC 360

TACCTCTGTG GGGCCATCTC CCTGGCCCCC AAGGCGCAGA TCAAAGAGAG CCTGCGGGCA 420

GAGCTCAGGG TGACAGAGAG AAGGGCAGAA GTGCCCACAG CCCACCCCAG CCCCTCACCC 480

AGGTCAGCCG GCCAGTTCCA AACCCTGGTG GTTGGTGTCG TGGGCGGCCT GCTGGGCAGC 540

CTGGTGCTGC TAGTCTGGGT CCTGGCCGTC ATCTGCTCCC GGGCCGCACG AGGGACAATA 600

GGAGCCAGGC GCACCGGCCA GCCCCTGAAG GAGGACCCCT CAGCCGTGCC TGTGTTCTCT 660

GTGGACTATG GGGAGCTGGA TTTCCAGTGG CGAGAGAAGA CCCCGGAGCC CCCCGTGCCC 720

TGTGTCCCTG AGCAGACGGA GTATGCCACC ATTGTCTTTC CTAGCGGAAT GGGCACCTCA 780

TCCCCCGCCC GCAGGGGCTC AGCTGACGGC CCTCGGAGTG CCCAGCCACT GAGGCCTGAG 840

GATGGACACT GCTCTTGGCC CCTC 864

(2) INFORMATION FOR SEQ ID NO:3:

(i) SEQUENCE CHARACTERISTICS:
(A) LENGTH: 921 base pairs
(B) TYPE: nucleic acid
(C) STRANDEDNESS: single
(D) TOPOLOGY: linear

(ii) MOLECULE TYPE: cDNA to mRNA

(xi) SEQUENCE DESCRIPTION: SEQ ID NO:3:

CACTCTGGTG GGGCTGCTCC AGGCATGCAG ATCCCACAGG CGCCCTGGCC AGTCGTCTGG 60

GCGGTGCTAC AACTGGGCTG GCGGCCAGGA TGGTTCTTAG ACTCCCCAGA CAGGCCCTGG 120

AACCCCCCCA CCTTCTCCCC AGCCCTGCTC GTGGTGACCG AAGGGGACAA CGCCACCTTC 180

ACCTGCAGCT TCTCCAACAC ATCGGAGAGC TTCGTGCTAA ACTGGTACCG CATGAGCCCC 240

AGCAACCAGA CGGACAAGCT GGCCGCCTTC CCCGAGGACC GCAGCCAGCC CGGCCAGGAC 300

TGCCGCTTCC GTGTCACACA ACTGCCCAAC GGGCGTGACT TCCACATGAG CGTGGTCAGG 360

GCCCGGCGCA ATGACAGCGG CACCTACCTC TGTGGGGCCA TCTCCCTGGC CCCCAAGGCG 420

CAGATCAAAG AGAGCCTGCG GGCAGAGCTC AGGGTGACAG AGAGAAGGGC AGAAGTGCCC 480

ACAGCCCACC CCAGCCCCTC ACCCAGGTCA GCCGGCCAGT TCCAAACCCT GGTGGTTGGT 540

GTCGTGGGCG GCCTGCTGGG CAGCCTGGTG CTGCTAGTCT GGGTCCTGGC CGTCATCTGC 600

TCCCGGGCCG CACGAGGGAC AATAGGAGCC AGGCGCACCG GCCAGCCCCT GAAGGAGGAC 660

CCCTCAGCCG TGCCTGTGTT CTCTGTGGAC TATGGGGAGC TGGATTTCCA GTGGCGAGAG 720

AAGACCCCGG AGCCCCCCGT GCCCTGTGTC CCTGAGCAGA CGGAGTATGC CACCATTGTC 780

TTTCCTAGCG GAATGGGCAC CTCATCCCCC GCCCGCAGGG GCTCAGCTGA CGGCCCTCGG 840

AGTGCCCAGC CACTGAGGCC TGAGGATGGA CACTGCTCTT GGCCCCTCTG ACCGGCTTCC 900

TTGGCCACCA GTGTTCTGCA G 921

(2) INFORMATION FOR SEQ ID NO:4:

(i) SEQUENCE CHARACTERISTICS:
(A) LENGTH: 921 base pairs
(B) TYPE: nucleic acid
(C) STRANDEDNESS: single
(D) TOPOLOGY: linear

(ii) MOLECULE TYPE: cDNA to mRNA

(vi) ORIGINAL SOURCE:
(A) ORGANISM: Homo sapiens
(H) CELL LINE: YTC3

(ix) FEATURE:
(A) NAME/KEY: CDS
(B) LOCATION: 25..888
(C) IDENTIFICATION METHOD: P

(ix) FEATURE:
(A) NAME/KEY: sig_peptide
(B) LOCATION: 25..84
(C) IDENTIFICATION METHOD: S

(ix) FEATURE:
(A) NAME/KEY: mat_peptide
(B) LOCATION: 85..888
(C) IDENTIFICATION METHOD: S

(xi) SEQUENCE DESCRIPTION: SEQ ID NO:4:
CACTCTGGTG GGGCTGCTCC AGGC ATG CAG ATC CCA CAG GCG CCC TGG CCA 51
GTC GTC TGG GCG GTG CTA CAA CTG GGC TGG CGG CCA GGA TGG TTC TTA 99
GAC TCC CCA GAC AGG CCC TGG AAC CCC CCC ACC TTC TCC CCA GCC CTG 147
CTC GTG GTG ACC GAA GGG GAC AAC GCC ACC TTC ACC TGC AGC TTC TCC 195
AAC ACA TCG GAG AGC TTC GTG CTA AAC TGG TAC CGC ATG AGC CCC AGC 243
AAC CAG ACG GAC AAG CTG GCC GCC TTC CCC GAG GAC CGC AGC CAG CCC 291
GGC CAG GAC TGC CGC TTC CGT GTC ACA CAA CTG CCC AAC GGG CGT GAC 339
TTC CAC ATG AGC GTG GTC AGG GCC CGG CGC AAT GAC AGC GGC ACC TAC 387
CTC TGT GGG GCC ATC TCC CTG GCC CCC AAG GCG CAG ATC AAA GAG AGC 435
CTG CGG GCA GAG CTC AGG GTG ACA GAG AGA AGG GCA GAA GTG CCC ACA 483
GCC CAC CCC AGC CCC TCA CCC AGG TCA GCC GGC CAG TTC CAA ACC CTG 531
GTG GTT GGT GTC GTG GGC GGC CTG CTG GGC AGC CTG GTG CTG CTA GTC 579
TGG GTC CTG GCC GTC ATC TGC TCC CGG GCC GCA CGA GGG ACA ATA GGA 627
GCC AGG CGC ACC GGC CAG CCC CTG AAG GAG GAC CCC TCA GCC GTG CCT 675
GTG TTC TCT GTG GAC TAT GGG GAG CTG GAT TTC CAG TGG CGA GAG AAG 723
ACC CCG GAG CCC CCC GTG CCC TGT GTC CCT GAG CAG ACG GAG TAT GCC 771
ACC ATT GTC TTT CCT AGC GGA ATG GGC ACC TCA TCC CCC GCC CGC AGG 819
GGC TCA GCT GAC GGC CCT CGG AGT GCC CAG CCA CTG AGG CCT GAG GAT 867
GGA CAC TGC TCT TGG CCC CTC TGACCGGCTT CCTTGGCCAC CAGTGTTCTG 918
CAG 921

## Description

### Field of the Invention

The present invention is related to a novel peptide cell death and DNAs encoding it.

### Background

Developmentally and physiologically controlled cell deaths can be observed in almost all tissues of various species of animals. Such cell deaths are generally considered 'programmed' and distinguished from 'accidental' deaths that occur by pathological mechanisms. Most of the cells undergoing programmed death have been shown to require de novo synthesis of RNA and protein.

These facts suggest that at least a few genes, if not specified ones, must be expressed to cause programmed cell death.

The term 'apoptosis', on the other hand, is used to describe the morphological characteristics of a class of cell death. In cells dying by apoptosis, the chromatin condenses around the periphery of the nucleus, while the mitochondria and other organelles are unaffected. A unique biochemical feature of apoptotic cells includes fragmentation of DNA into oligonucleosomal pieces. In mammals, apoptosis is often associated with programmed cell death morphologically and biochemically, but some of the cells undergoing programmed death apparently do not show the characteristic features of apoptosis. In addition, there are apoptotic cell deaths that can be induced in the absence of any protein synthesis.

Thus, it is important to note that apoptosis is not synonymous with programmed cell death.

Recently, if has been apparent that bcl-2 which is a oncogene and in mortalized B cells by protection the cell death, it was shown the importance to control the cell death.

### Related Arts

From now, certain peptides which are related to programmed cell death were reported. In such peptides, one of the representative is Fas antigen (Itoh, N. et al., Cell, 66, 233 (1991)).

Human Fas antigen is a polypeptide consisting from 335 amino acids, having signal peptide consisting 16 hydrophobic amino acids N-terminal and it was considered that its mature protein have a structure divided to extracellular domain (157 amino acids), transmembrane region (17 amino acids) and cytoplasmic domain (145 amino acids). And it was thought that Fas antigen had a function of receptor to a factor (ligand) inducing cell death.

### Purpose of the Invention

The purpose of the present invention is to find novel polypeptide which is alternative from polypeptides represented by Fas antigen.

In this invention, gene deeply related to programmed cell death have been isolated, its nucleotide sequence have been decided and its amino acid sequence have been deduced. And the present inventors have succeeded to find a quite novel polypeptide and DNA encoding it, and then completed the present invention.

To isolate a gene deeply related to programmed cell death in human, mouse PD-1 which obtained from mouse T cell hybridoma 2B 4.11 (Japanese Patent Kokai 5-336973) was used as probe.

There was no polypeptides having amino acid sequence which is identical to or has high homology to that of the polypeptide of the present invention except for mouse PD-1, when amino acid sequences of the polypeptide identified in the present invention was compared by a computer program for all known sequences in data base of National Biomedical Research Foundation. Needless to say, it was confirmed that the polypeptide has no homology to Fas antigen.

### Constitution of the Invention

The present invention is related to polypeptide which is deeply related to programmed cell death (abbreviated human PD-1 hereafter).

The present invention is concerned with a polypeptide having the amino acid shown in SEQ. ID. No. 1, in substantially purified form, a homologue thereof or a fragment of the sequence or homologue of a fragment, and DNA encoding such a polypeptide. More particularly, the present invention is related to DNA having the nucleotide sequence shown in SEQ. ID. No. 2 or 3, and DNA having a fragment which is selectively hybridizing to nucleotide sequence shown in SEQ. ID. No. 2 or 3.

The present invention is related to:
(1) a polypeptide having an amino acid sequence shown in SEQ. ID. No. 1,
(2) a DNA encoding the polypeptide described above (1),
(3) a DNA having a nucleotide sequence shown in SEQ. ID. No. 2, and
(4) a DNA having a nucleotide sequence shown in SEQ. ID. No. 3.

A polypeptide of SEQ. ID. No. 1 in substantially purified form will generally comprise the polypeptide in a preparation in which more than 90%, e.g. 95%, 98% or 99% of the polypeptide in the preparation is that of the SEQ. ID. No. 1.

A polypeptide homologue of the SEQ. ID. No. 1 will be generally at least 70%, preferably at least 80 or 90% and more preferably at least 95% homologous to the polypeptide of SEQ. ID. No. 1 over a region of at least 20, preferably at least 30, for instance 40, 60 or 100 more contiguous amino acids. Such polypeptide homologues will be referred to below as a polypeptide according to the invention.

Generally, fragments of SEQ. ID. No. 1 or its homologues will be at least 10, preferably at least 15, for example 20, 25, 30, 40, 50 or 60 amino acids in length, and are also encompassed by the term "a polypeptide according to the invention" as used herein.

A DNA capable of selectively hybridizing to the DNA of SEQ. ID. No. 2 or 3 will be generally at least 70%, preferably at least 80 or 90% and more preferably at least 95% homologous to the DNA of SEQ. ID. No. 2 or 3 over a region of at least 20, preferably at least 30, for instance 40, 60 or 100 or more contiguous nucleotides. Such DNA will be encompassed by the term "DNA according to the invention".

Fragments of the DNA of SEQ. ID. No. 2 or 3 will be at least 15, preferably at least 20, for example 25, 30 or 40 nucleotides in length, and are also encompassed by the term "DNA according to the invention" as used herein.

A further embodiment of the invention provides replication and expression vectors comprising DNA according to the invention. The vectors may be, for example, plasmid, virus or phage vectors provided with an origin of replication, optionally a promoter for the expression of the said DNA and optionally a regulator of the promoter. The vector may contain one or more selectable marker genes, for example a anpicillin resistance gene. The vector may be used in vitro, for example of the production of RNA corresponding to the DNA, or used to transfect or transform a host cell.

A further embodiment of the invention provides host cells transformed or transfected with the vectors for the replication and expression of DNA according to the invention, including the DNA SEQ. ID. No. 2 or 3 or the open reading frame thereof. The cells will be chosen to be compatible with the vector and may for example be bacterial, yeast, insect or mammalian.

A further embodiment of the invention provides a method of producing a polypeptide which comprises culturing host cells of the present invention under conditions effective to express a polypeptide of the invention. Preferably, in additions such a method is carried out under conditions in which the polypeptide of the invention is expressed and then produced from the host cells.

DNA according to the invention may also be inserted into the vectors described above in an antisense orientation in order to proved for the production of antisense RNA. Antisense RNA may also be produced by synthetic means. Such antisense RNA may be used in a method of controlling the levels of a polypeptide of the invention in a cell.

The invention also provides monoclonal or polyclonal antibodies to a polypeptide according to the invention. The invention further provides a process for the production of monoclonal or polyclonal antibodies to the polypeptides of the invention. Monoclonal antibodies may be prepared by conventional hybridoma technology using a polypeptide of the invention or a fragment thereof, as an immunogen. Polyclonal antibodies may also be prepared by conventional means which comprise inoculating a host animal, for example a rat or a rabbit, with a polypeptide of the invention and recovering immune serum.

The present invention also provides pharmaceutical compositions containing a polypeptide of the invention, or an antibody thereof, in association with a pharmaceutically acceptable diluent and/or carrier.

The polypeptide of the present invention includes that which a part of their amino acid sequence is lacking (e.g., a polypeptide comprised of the only essential sequence for revealing a biological activity in an amino acid sequence shown in SEQ. ID. No.1), that which a part of their amino acid sequence is replaced by other amino acids (e.g., those replaced by an amino acid having a similar property) and that which other amino acids are added or inserted into a part of their amino acid sequence, as well as those having the amino acid sequence shown in SEQ. ID. No. 1.

As known well, there are one to six kinds of codon as that encoding one amino acid (for example, one kind of codon for Methioine (Met), and six kinds of codon for leucine (Leu) are known). Accordingly, the nucleotide sequence of DNA can be changed in order to encode the polypeptide having the same amino acid sequence.

The DNA of the present invention, specified in (2) includes a group of every nucleotide sequences encoding polypeptides (1) shown in SEQ. ID. No. 1 . There is a probability of improving a yield of production of a polypeptide by changing a nucleotide sequence.

The DNA specified in (3) is the embodiment of DNA shown in (2), and is sequence in the natural form.

The DNA shown in (4) indicates the sequence of the DNA specified in (3) with a non-translational region.

The DNA of the present invention may be obtained by gene recombination, chemical synthesis or known methods for the skilled in the arts.

Human PD-1 includes a series of polypeptides which are deferent from Fas antigen in structural feature and commonly in mammals. That is, PD-1 of the present invention includes human PD-1 declared in the present invention and PD-1 of the other mammals which have high homology (it means immunological equivalent which can be cross-reacted to human PD-1 antigen).

The structural feature of human PD-1 is as follows:

Human PD-1 is predicted a membrane binding type protein consisting with 288 amino acids. It contains two hydrophobic regions, one at the N terminus and the other in the middle, which are likely to serve as a signal peptide and a transmembrane segment, respectively.

Comparison of the N-terminal sequence of the PD-1 protein with typical signal peptide cleavage sites suggests that the signal peptide would be from Met1 to Arg20. Thus, the predicted mature form of the PD-1 protein would contain 268 amino acids and consists of an extracellular domain (147 amino acids), a transmembrane region (27 amino acids) and a cytoplasmic domain (94 amino acids). Four potential N-glycosylation sites are found in the putative extracellular domain.

Comparison of the amino acid sequence of the PD-1 protein with all sequences registered in the National Biomedical Research Foundation data base revealed that the extracellular domain of the PD-1 protein is homologous to some members of the immunoglobulin superfamily. Immunogloblin domains have been classified into V, C1 and C2 sets based on the conserved amino acid patterns and the number of antiparallel beta-strands. The 68 amino acid residue between two cystein residues (Cys54 and Cys123) in PD-1 bear resemblance to a disulfidelinked immunogloblin domain of the V-set sequences. In addition, all of the four amino acid residues characteristic of many V-set sequences are also conserved in PD-1 (Arg94, Phe95, Asp117 and Gly119).

The cytoplasmic domain of the predicted PD-1 protein contains a variant form of the consensus sequence (Asp/Glu-X8-Asp/Glu-X2-Tyr-X2-Leu/Ile-X7-Tyr-X2-Leu/Ile) found in the cytoplasmic tails of most of the polypeptides associated with antigen receptors and Fc receptors. It was recently shown that one signal unit of this consensus sequence is sufficient to transduce signals.

It is thought that PD-1 of other mammals would be similar to human PD-1 in structural feature, whethr or not, number or kinds of its amino acid would be different in its sequence.

### Preparation

DNA encoding human PD-1 of the present invention may be prepared by the following method.

Once the nucleotide sequences shown in SEQ. ID. Nos. 2 and 3 are determined, DNA of the present invention may be obtained by chemical synthesis, by PCR method or by hybridization making use of a fragment of DNA of the present invention, as probe. Furthermore, DNA of the present invention may be obtained in a desired amount by transforming with a vector DNA inserted a DNA of the present invention into a proper host, followed by culturing the transformant.

The PD-1 polypeptides of the present invention (shown in SEQ. ID. No. 1) may be prepared by:
(1) isolating and purifying from an organism or a cultured cell,
(2) chemically synthesizing, or
(3) using a skill of biotechnology,
preferably, by the method described in (3).

Examples of expression system when preparing a polypeptide by using a skill of biotechnology, is, for example, the expression system of bacteria, yeast, insect cell and mammalian cell.

For example, the expression in E. coli may be carried out by adding the initiation codon (ATG) to 5' end of a DNA encoding mature peptide, connecting the DNA thus obtained to the downstream of a proper promoter (e.g., trp promoter, lac promoter, λ PL promoter, T7 promoter etc.), and then inserting it into a vector (e.g., pBR322, pUC18, pUC19 etc.) which functions in an E. coli strain to prepare an expression vector.

Then, an E. coli strain (e.g., E. coli DH1 strain, E. coli JM109 strain, E. coli HB101 strain, etc.) which is transformed with the expression vector thus obtained may be cultured in a proper medium to obtain the desired polypeptide. When a signal peptide of bacteria (e.g., signal peptide of pel B) is utilized, the desired polypeptide may be also released in periplasm. Furthermore, a fusion protein with other polypeptide may be also produced easily.

Furthermore, the expression in a mammalian cell may be carried out, for example, by inserting the total DNA encoding PD-1 into the downstream of a proper promoter (e.g., SV40 promoter, LTR promoter, metallothionein promoter etc.) in a proper vector (e.g., retrovirus vector, papilloma virus vector, vaccinia virus vector, SV40 vector, etc.) to obtain an expression vector, and transforming a proper mammalian cell (e.g., monkey COS-7 cell, Chinese hamster CHO cell, mouse L cell etc.) with the expression vector thus obtained, and then culturing the transformant in a proper medium to get a desired polypeptide in the culture medium. The polypeptide thus obtained may be isolated and purified by conventional biochemical methods.

DNA encoding PD-1 gene obtained by the present invention may be used for the isolation of PD-1 gene of other animals as probe.

The cDNA having a nucleotide sequence shown in SEQ. ID. No. 3 may be prepared according to the following methods, that is:
(i) by isolating mRNA from a cell line which products the polypeptide of the present invention (e.g., human esophageal cancer cell line),
(ii) by preparing first strand (single stranded DNA) from mRNA thus obtained, followed by preparing second strand (double stranded DNA) (synthesis of cDNA),
(iii) by inserting cDNA thus obtained into a proper phage vector,
(iv) by transforming host cells with the recombinant DNA thus obtained (preparation of cDNA library),
(v) by screening with plaque hybridization from cDNA library thus obtained with cDNA of mouse PD-1 as probe,
(vi) by preparing phage DNA from positive clone obtained, subcloning cDNA released into plasmid vector, preparing restriction enzyme map, and
(vii) by deciding sequence of each restriction enzyme fragment, and by obtaining the full sequence of complete length by combining them.

Explained in detail, step (i) may be carried out in accordance with the method of Okayama, H. et al. (described in Methods in Enzymology, vol. 154, p 3, 1987) from human cell line after stimulation by a proper stimulant (e.g., IL-1 etc.) or without stimulation. Examples of the cells which product the polypeptide of the present invention is preferably human cell line YTC3.

Steps (ii), (iii) and (iv) are a series of steps for preparing cDNA library, and may be carried out in accordance with the method of Gubler & Hoffman (Gene, vol. 25, pp. 263, 1983) with a slight modification. As examples of the plasmid vector used in the step (iii), many plasmid vectors (e.g., pBR 322, pBluescript II) and phage vectors (e.g., λ gt10, λ DASH II) are known, and phage vector λ gt10 (43.3 kbp;Stratagene) may be preferably used.

As host cell used in step (iv), E. Coli NM514 (Stratagene) may be preferably used.

The steps (v) and (vi) may be carried out in accordance with the method described in Molecular Cloning (Sambrook, J., Fritsh, E. F., and Maniatis, T, Cold Spring Harbor Laboratory Press (1989)).

The step (vii) may be carried out in accordance with the method described in Molecular Cloning (written by Sambrook, J., Fritsch, E. F. and Maniatis, T., published by Cold Spring Harbor Laboratory Press in 1989).

The sequencing in the step (vii) may be carried out in accordance with the method of Maxam-Gilbert or the dideoxy termination method.

It is necessary to examine whether or not the cDNA thus obtained codes complete or almost complete length. The confirmation may be carried out by Northern analysis with the said cDNA as probe (see Molecular Cloning described before). It is thought that cDNA is almost complete length, if length of cDNA is almost same length of mRNA obtained in the hybridizing band.

DNA or DNA fragments encoding PD-1 gene may be used for detection of PD-1 gene as probe or a primer and thereby, and may be utilized for the purpose of investigating the relationship between the said polypeptide and protection mechanism in living organism, immunological function or diseased like tumour, or for the purpose of diagnosing diseases, and the like.

The DNA of the present invention may be utilized as an important and essential template in preparing by conventional gene recombination the PD-1 polypeptide, polypeptide fragment thereof or derivatives thereof which are expected to possess various use.

It is expected that the polypeptide, fragment polypeptides thereof or derived polypeptides thereof may be used for the treatment of infections, depression or acceleration of immunological function or tumour.

Further, polyclonal and monoclonal antibody against the polypeptide or polypeptide fragments of the present invention can be prepared by conventional method, and they can be used to quantitate the said polypeptide in organism, and thereby, may be utilized for the purpose of investigating the relationship between the said polypeptide and diseases, or for the purpose of diagnosing diseases, and the like. The said monoclonal antibody per se, chimeric antibody against human antibody may be used for the treating agent.
Polyclonal and monoclonal antibody thereof may be prepared by conventional methods by using the said polypeptide or the fragment thereof as an antigen.

### Application for Pharmaceuticals

For the purpose of the present invention, the polypeptide of the present invention may be normally administered systemically or partially, usually by oral or parenteral administration, preferably orally, intravenously or intraventricularly.

The doses to be administered are determined depending upon age, body weight, symptom, the desired therapeutic effect, the route of administration, and the duration of the treatment etc. In the human adult, the doses per person per dose are generally between 100 µg and 100 mg, by oral administration, up to several times per day, and between 10 µg and 100 mg, by parenteral administration up to several times per day.

As mentioned above, the doses to be used depend upon various conditions. Therefore, there are cases in which doses lower than or greater than the ranges specified above may be used.

Administration of the compounds of the present invention, may be as solid compositions, liquid compositions or other compositions for oral administration, as injections, liniments or suppositories etc. for parenteral administration.

Solid compositions for oral administration include compressed tablets, pills, capsules, dispersible powders, granules. Capsules include soft capsules and hard capsules.

In such compositions, one or more of the active compound(s) is or are admixed with at least one inert diluent (such as lactose, mannitol, glucose, hydroxypropyl cellulose, microcrystalline cellulose, starch, polyvinylpyrrolidone, magnesium metasilicate aluminate, etc.). The compositions may also comprise, as is normal practice, additional substances other than inert diluents: e.g. lubricating agents (such as magnesium stearate etc.), disintegrating agents (such as cellulose calcium glycolate, etc.), stabilizing agents (such as human serum albumin, lactose etc.), and assisting agents for dissolving (such as arginine, asparaginic acid etc.).

The tablets or pills may, if desired, be coated with a film of gastric or enteric material (such as sugar, gelatin, hydroxypropyl cellulose or hydroxypropylmethyl cellulose phthalate, etc.), or be coated with more than two films. And further, coating may include containment within capsules of absorbable materials such as gelatin.

Liquid compositions for oral administration include pharmaceutically-acceptable emulsions, solutions, syrups and elixirs. In such compositions, one or more of the active compound(s) is or are contained in inert diluent(s) commonly used in the art (purified water, ethanol etc.). Besides inert diluents, such compositions may also comprise adjuvants (such as wetting agents, suspending agents, etc.), sweetening agents, flavouring agents, perfuming agents, and preserving agents.

Other compositions for oral administration included spray compositions which may be prepared by known methods and which comprise one or more of the active compound(s). Spray compositions may comprise additional substances other than inert diluents: e.g. stabilizing agents (sodium sulfite etc.), isotonic buffer (sodium chloride, sodium citrate, citric acid, etc.). For preparation of such spray compositions, for example, the method described in the United States Patent No. 2,868,691 or 3,095,355 (herein incorporated in their entireties by reference) may be used.

Injections for parenteral administration include sterile aqueous or non-aqueous solutions, suspensions and emulsions. In such compositions, one or more active compound(s) is or are admixed with at least one inert aqueous diluent(s) (distilled water for injection, physiological salt solution, etc.) or inert non-aqueous diluents(s)(propylene glycol, polyethylene glycol, olive oil, ethanol, POLYSOLBATE 80 TM , etc.).

Injections may comprise additional other than inert diluents: e.g. preserving agents, wetting agents, emulsifying agents, dispersing agents, stabilizing agent (such as human serum albumin, lactose, etc.), and assisting agents such as assisting agents for dissolving (arginine, asparaginic acid, etc.).

They may be sterilized for example, by filtration through a bacteria-retaining filter, by incorporation of sterilizing agents in the compositions or by irradiation. They may also be manufactured in the form of sterile solid compositions, for example, by freeze-drying, and which can be dissolved in sterile water or some other sterile diluents for injection immediately before use.

Other compositions for parenteral administration include liquids for external use, and endermic liniments (ointment, etc.), suppositories for rectal administration and pessaries which comprise one or more of the active compound(s) and may be prepared by known methods.

### Examples

The following examples are illustrated, but not limit, the present invention.

### Example 1: Cell culture

The human cell lines (CESS, HPB-ALL, Jarkat, TC3, CCRF-CEM, JM and MOLT-4F) were cultured in RPMI 1640 (Gibco) supplemented with 10% heat-inactivated fetal calf serum, 2mM glutamine, 50 µM 2-mercaptoethanol, 100U/ml penicillin and 100 µg/ml streptmycin.

### Example 2 : Northern blot analysis

Total RNA was prepared from indicated cell lines by extraction with guanidium isothiocyanate method. (see molecular cloning described before), and poly (A)⁺ RNA was isolated from the total RNA by oligotex-dT 30 〈super〉 (Daiichi Chemical Co.). 3 µg of poly (A)⁺ RNA was separated on a 1.2% formaldehydeagarose gel, and transferred to a nylon membrane ( Biodyne A, Japan Genetic). The filter was baked at 80 °C for 2 hrs. Random priming was carried out to the Eco-RI fragment (1kb) containing the coding region of mouse PD-1, with ³²P to prepare as probe. Specific activity of this probe was about 9 x 10⁸ d.p.m./µg. Hybridization was carried out in 10 x Denhardt's, 1M NaCl, 50mM Tris (pH 7.5), 10 mM EDTA, 1% SDS and 1 mg/ml sonicated salmon sperm DNA at 65 °C for 15 hrs. The filter was washed in 1 x SSC, 0.1% SDS at 65 °C for 10 mins. Hybridization signal (2.3 kb) was observed from lymphocyte cell line YTC3 by autoradiography.

### Example 3 : Construction of cDNA library and cloning of human PD-1 cDNA

cDNA library was constructed with 5 µg of poly (A)⁺ RNA extracted from YTC3 cell lines by using Time Saver cDNA Synthesis Kit (Pharmacia). Synthesis of first strand cDNA was carried out with oligo dT primer. Double stranded cDNA which was ligated EcoRI-NotI adopter, was cloned into λ gt 10 vector, and packaged into phage (Gigapack II Gold, Stratagene). Phage was plated on a lawn of E. Coli NM514. Phage DNA was transfected to duplicated library filters from each plate. The filters were baked at 80 °C for 2 hrs and hybridized at 60 °C for 15 hrs. The mouse PD-1 coding region (1 kb) excised with EcoRI from Bluescript SK plasmid vector ( Stratagene ) was used as probe. The filter was washed with 1 x SSC and 0.1% SDS at 60 °C for 10 mins. 51-Positive signals were observed from 1.2 x 10⁶ phages by autoradiography. These clones were purified to single. Further analysis was carried out about 23 clones picked up, the longest cDNA insert observed was 2.1 kb. This result coincided to the result of Southern Blot analysis.

### Example 4 : Sequencing of DNA

The cDNA inserts isolated from human cDNA library were subcloned into Bluescript SK plasmid vectors ( Stratagene), and sequenced by the dideoxynucleotide chain termination method ( Sanger et al., 1977) using a modified T7 DNA polymerase ( United States Biochemical ) and [α-³²P]dCTP ( 3000 Ci/mmol, Amersham). Specific primer of Bluescript plasmid was used as a sequencing primer. Nucleotide sequencing was carried out by fully sequencing for both strands of the cDNA. From the result, nucleotide sequence shown in seq. No. 2 was obtained. Deduced peptide sequence ( shown in SEQ. ID. No. 1) was determined from the nucleotide sequence. Total number of deduced amino acid is 288, and the number is the same as that of mouse PD-1. Homology of was found about 60 % each other.

### Example 5 : Southern Blotting

Genomic DNA were isolated from kinds of animal cells by conventional method ( see Molecular Cloning described before ). DNAs were digested by EcoRI, BamHI or hind III followed according to the manufactures-recommendation, isolated by electrophoresis ( 100V, 0.8% agarose gel, TEA buffer). DNA fragments were washed with 0.25N HCl for 10 mins, denatured with 0.2N NaOH / 0.6M NaCl for 30 mins, neutralized with 0.6M NaCl / 0.2M Tris (pH 7.5 ) for 1 hr., transferred to nylon membrane (Bidyne A) which is used standard Southern procedure. The filter were baked for 2 hrs.

Random priming was carried out to the EcoRI-StuI fragment (900 bp) containing the coding region of human PD-1, ³²P to prepare as probe. Specific activity of this probe was about 9 x 10⁸ d.p.m./µg. Hybridization was carried out in 10 x Denhardt's, 1M NaCl, 50mM Tris (pH 7.5), 10 mM EDTA, 1% SDS and 1mg/ml sonicated salmon sperm DNA at 65 °C for 10 mins. The filter was washed in 1 x SSC, 0.1% SDS at 65 °C for 10 mins. Only one band was detected by autoradiography when the clone was cut with any enzyme, it was found that human PD-1 gene exists as single copy.

Southern hybridization was carried out with genomic DNA of kinds of animals by the same condition (hybridization and washing) described in example 2, using EcoRI fragment (1kb) containing coding region of mouse PD-1 as probe. Hybridization signals were detected from only genomic DNA of mouse and human, and were not detected from genomic DNA of Drosophila, Xenopus and rabbit.

### Example 6 : Isolation of genomic clone of human PD-1

A genomic DNA library from esophageal cancer cell line was constructed in the λ DASH II vector via Sau3AI partial digestion and ligation into the BamHI site (obtained from Dr. Nishiyama, 1st Dept. of Pathology, School of Medicine, Kyoto University). The human PD-1 gene was isolated from this library by hybridization with the human PD-1 total cDNA excised with EcoRI digestion from Bluescript SK vector. The probe was labeled with ³²P by random priming. Two positive clones was isolated and purified from 1 x 10⁶ phage plaque, digested by several restriction enzymes, and analyzed by Southern hybridization using the same probe. From CISS (chromosomal in situ suppression) , it was found that human PD-1 gene was mapped on 2q37.3.

## Claims

1. A polypeptide having the amino acid sequence shown in SEQ. ID. No. 1 in substantially purified form, a homologue thereof or a fragment of the sequence or homologue of a fragment.

2. A polypeptide according to claim 1 having the amino acid sequence shown in SEQ. ID. No. 1.

3. DNA encoding a polypeptide according to claim 1.

4. DNA according to claim 3 having the nucleotide sequence shown in SEQ. ID. No. 2 or a fragment thereof capable of selectively hybridizing to SEQ. ID. No. 2.

5. DNA according to claim 3 having the nucleotide sequence shown in SEQ. ID. No. 3 or a fragment thereof capable of selectively hybridizing to SEQ. ID. No. 3.

6. A replication and expression vector comprising DNA according to any one of claims 3 to 5.

7. Host cells transformed or tranfected with a replication and expression vector according to claim 6.

8. A method of producing a polypeptide which comprises culturing host cells according to claim 7 under conditions effective to express a polypeptide according to claim 1 or 2.

9. A monoclonal or polyclonal antibody to a polypeptide according to claim 1 or 2.

10. A pharmaceutical composition containing a polypeptide according to claims 1 or 2 or an antibody according to claim 9 in association with a pharmaceutically acceptable diluent and/or carrier.
